# EUROPÄISCHE PATENTANMELDUNG

(11) **EP 4 793 269 A1**
(43) Veröffentlichungstag der Anmeldung: **19.08.2026**
(21) Anmeldenummer: 25220774.1
(22) Anmeldetag: 04.12.2025
(51) Int. Cl.: C07C 265/14, C07C 263/20

(54) **DESTILLATION VON ISOCYANATEN MIT HOHEM SIEDEPUNKT**

(30) Priorität: 14.02.2025 EP 25158034
(71) Anmelder: Covestro Deutschland AG, 51373 Leverkusen (DE)
(72) Erfinder: Merkel, Michael, 40223 Düsseldorf (DE); Schneider, Ralf, 51375 Leverkusen (DE); Stolz, Sebastian, 51061 Köln (DE)
(74) Vertreter: Levpat

(57) **Zusammenfassung**

Die Erfindung betrifft ein Verfahren zur destillativen Reinigung von Isocyanaten mit einem hohen Schmelz- und Siedepunkt. Durch Verwendung einer Druckregelung im Verdampfungsraum kann ein Kühlmedium im Verdampfer-Kondensator für die destillative Reinigung verschiedener Isocyanate in derselben Vorrichtung verwendet werden.

## Beschreibung

Die vorliegende Erfindung betrifft ein Verfahren zur destillativen Reinigung von den beiden Isocyanaten 1,5-Naphthalindiisocyanat oder p-Phenylendiisocyanat, die sich durch hohe Schmelzpunkte auszeichnen. Außerdem betrifft die Erfindung eine Vorrichtung zur destillativen Reinigung dieser Isocyanate.

Die Destillation von 1,5-Naphthalindiisocyanat oder p-Phenylendiisocyanat stellt eine besondere verfahrenstechnische Herausforderung dar. Aufgrund der Fähigkeit der Isocyanate, bei erhöhten Temperaturen mit sich selbst zu reagieren, muss die Destillation bei sehr niedrigem Druck erfolgen. Auf diese Weise lässt sich die Bildung von höhermolekularen Rückständen möglichst gering halten. Dies wiederum führt dazu, dass auch die Kondensation des Destillats bei verhältnismäßig niedrigen Temperaturen durchgeführt werden muss. Temperaturen unterhalb des Schmelzpunkts des bei der Destillation verdampften Isocyanats führen dabei zur Kondensation des Isocyanats als Feststoff, was verfahrenstechnische Herausforderungen mit sich bringt. Zu hohe Temperaturen führen hingegen zu Nebenreaktionen und zu einer unvollständigeren Kondensation, welche wiederum einen erhöhten Aufwand für die Aufbereitung des Destillationsabgases nach sich zieht.

Die EP 4038049 A1 beschreibt ein Verfahren zur Vakuumdestillation von Isocyanaten, wobei das Vakuum durch einen Flüssigkeitsringverdichter erzeugt wird. 1,5-Naphthalindiisocyanat und p-Phenylendiisocyanat werden zwar in einer Liste von für das Verfahren geeigneten Isocyanaten aufgezählt, es wird jedoch nicht darauf eingegangen, wie die Kondensation dieser besonderen Isocyanate angesichts ihrer hohen Schmelzpunkte auszuführen ist. In der Beschreibung zum Stand der Technik wird ausgeführt, dass viele technisch relevante Isocyanate nach Kondensation als flüssiger Seitenstrom aus der Reindestillations-Kolonne entnommen werden. Ob ein solches Verfahren auch für die speziellen Isocyanate 1,5-Naphthalindiisocyanat und p-Phenylendiisocyanat existiert, ist dem nicht zu entnehmen und es fehlt jeglicher Hinweis, wie es im Falle von hochschmelzenden Isocyanaten zu realisieren wäre.

Im Stand der Technik finden sich mehrere Dokumente, die offenbaren, wie 1,5-Naphthalindiisocyanat oder andere bei Raumtemperatur feste Diisocyanate aus Rückständen durch Destillation zurückgewonnen werden können. Dabei wird häufig ausgeführt, dass das aus dem Rückstand in die Gasphase überführte Diisocyanat als Feststoff kondensiert wird, wie z.B. in WO 2018134238 A1 beschrieben, oder dass die Kühlmitteltemperatur unterhalb des Schmelzpunktes des Diisocyanats liegt, was letztlich ebenfalls zu einer Kondensation als Feststoff führt, wie beispielsweise in der WO 2020016142 A1 oder der WO 2021148419 A1 beschrieben. Zum Austrag des so erhaltenen Feststoffs ist es üblich, den Kondensator abzuschmelzen, was entweder eine Prozessunterbrechung erfordert oder die parallele Installation von mindestens zwei Kondensatoren, die im Wechsel betrieben werden.

Als eine Lösung für dieses Problem beschreibt die EP 4121410 A1 einen aufwändigen Apparat mit einer rotierenden Kondensationswalze, von der das Produkt im Betrieb abgeschabt und als Feststoff ausgetragen wird.

Einen anderen Weg, der insbesondere für großtechnische Verfahren vorteilhaft ist, beschreibt die DE 2035731 A1. Dort wird für die Kondensation des verdampften Isocyanats ein Verdampfer-Kondensator eingesetzt. Dieser wird in den beiden Ausführungsbeispielen jeweils mit einem Kühlmedium befüllt, dessen Siedepunkt zwischen dem Schmelzpunkt und dem Kondensationspunkt des zu destillierenden Isocyanats bei Prozessbedingungen liegt. Für die destillative Reinigung von HDI mit einem Schmelzpunkt von -67 °C wird dabei siedendes Wasser und für die destillative Reinigung von 1,5-Naphthalindiisocyanat mit einer Schmelztemperatur von 127 °C bis 130 °C siedendes Chlorbenzol als Kühlmedium eingesetzt. Auf diese Weise kondensiert das jeweilige Isocyanat zunächst als Flüssigkeit, die vom Kondensator frei abläuft und ausgetragen werden kann. Nicht kondensierte Anteile des Isocyanats werden in einem nachgeschalteten Wäscher aus dem Destillationsabgas ausgewaschen. Nachteilig an diesem Verfahren ist der Umstand, dass ein geeignetes Kühlmedium gefunden werden muss. Dieses muss nicht nur einen passenden Siedepunkt aufweisen, es sollte zudem auch selbst thermisch stabil sein, ökologisch und toxikologisch möglichst unbedenklich sein, im Falle einer Innenleckage keine gefährlichen Reaktionen mit dem Isocyanat eingehen, kostengünstig verfügbar sein und sich gut in den Prozess und die mögliche Entsorgung integrieren lassen. Ein weiterer Nachteil an diesem Verfahren ist, dass es nicht flexibel genug ist, um auf Veränderungen im Prozess zu reagieren. Kommt es zum Beispiel zu einer Änderung des Drucks in der Destillationsapparatur oder zu einer Verschlechterung des Wärmeübergangs im Verdampfer-Kondensator, beispielsweise in Folge von Ablagerungen, kann die Betriebstemperatur des Kondensators nicht angepasst werden, um auf diese Veränderungen zu reagieren, da sie auf den Siedepunkt des jeweiligen Kühlmediums festgelegt ist. Bei Änderungen des Umgebungsdrucks kommt es hingegen ungewollt zu einer Änderung der Siedetemperatur des Kühlmediums, die sich wiederum unkontrolliert auf den Prozess auswirkt.

Aufgabe der Erfindung war es, ein verbessertes Verfahren zur Destillation der beiden bei hohen Temperaturen, oberhalb von 95°C und unterhalb von 160°C, schmelzenden Isocyanate 1,5-Naphthalindiisocyanat oder p-Phenylendiisocyanat zur Verfügung zu stellen, bei dem trotz der Verwendung eines Verdampfer-Kondensators auf Prozessveränderungen reagiert werden kann und bei dem die Kondensatortemperatur weniger vom verwendeten Kühlmedium abhängig ist. Das durch destillative Abtrennung gewonnene Isocyanat sollte bevorzugt möglichst frei von Verunreinigungen und sonstigen Fremdstoffen sein, so dass es möglichst ohne weitere Reinigungsprozesse als Edukt für nachfolgende Synthesen, beispielsweise Oligomer- oder Polymersynthesen, verwendet werden kann.

Diese Aufgabe wird gelöst werden durch ein Verfahren zur destillativen Reinigung von 1,5-Naphthalindiisocyanat oder p-Phenylendiisocyanat, umfassend die Schritte (i), (ii), (iii) und (iv) in der folgenden Reihenfolge
(i) Bereitstellen einer Isocyanat-Zusammensetzung, enthaltend 1,5-Naphthalindiisocyanat oder p-Phenylendiisocyanat,
(ii) zumindest teilweises Verdampfen des in der Isocyanat-Zusammensetzung enthaltenen 1,5-Naphthalindiisocyanats oder p-Phenylendiisocyanats unter Erhalt dieses Isocyanats im gasförmigen Zustand,
(iii) zumindest teilweise Kondensation und dadurch Erhalt eines Isocyanat-Kondensats des in Schritt (ii) in den gasförmigen Zustand überführten Isocyanats in einem Verdampfer-Kondensator unter Verwendung eines Kühlmediums, umfassend a) wenigstens einen Kondensationsraum zur Kondensation des in Schritt (ii) in den gasförmigen Zustand überführten Isocyanats und b) wenigstens einen mit dem Kondensationsraum im Wärmeaustausch stehenden Verdampfungsraum zur teilweisen Verdampfung des Kühlmediums und
(iv) Austragen des in Schritt (iii) erhaltenen flüssigen Isocyanat-Kondensats aus dem Kondensationsraum,
dadurch gekennzeichnet,
dass mit Hilfe einer Druckanpassungseinrichtung ein Druck P1 in dem wenigstens einen Verdampfungsraum eingestellt wird, bei dem sich für das Kühlmedium eine Siedetemperatur ergibt, die mindestens 3 K, bevorzugt mindestens 8 K, unterhalb der Kondensationstemperatur des zu reinigenden Isocyanats unter den im Kondensationsraum herrschenden Bedingungen, jedoch nicht mehr als 5 K unterhalb der Schmelztemperatur, bevorzugt oberhalb der Schmelztemperatur des zu reinigenden Isocyanats bei Normaldruck liegt,
wobei, wenn es sich bei dem Isocyanat um 1,5-Naphthalindiisocyanat handelt, in dem wenigstens einen Kondensationsraum, in dem die Kondensation, also die mindestens teilweise Kondensation, von gasförmigem Isocyanat aus Schritt (ii) erfolgt, ein Druck P2 im Bereich von 80 bis 1.000 Pa vorliegt,
und wobei, wenn es sich bei dem Isocyanat um p-Phenylendiisocyanat handelt, in dem wenigstens einen Kondensationsraum, in dem die Kondensation, also die mindestens teilweise Kondensation, von gasförmigem Isocyanat aus Schritt (ii) erfolgt, ein Druck P2 im Bereich von 1.000 bis 20.000 Pa vorliegt.

Für technisch bedeutsame Isocyanate wie 1,5-Naphthalindiisocyanat und p-Phenylendiisocyanat finden sich in der Literatur zuverlässige Angaben über deren Schmelztemperatur. Häufig wird jedoch ein Schmelzbereich angegeben, so beispielsweise ein Bereich von 127 °C bis 130 °C für 1,5-Naphthalindiisocyanat und ein Bereich von 96 °C bis 99 °C für p-Phenylendiisocyanat. Oberhalb dieses Bereichs ist davon auszugehen, dass das entsprechende Isocyanat bis zu seinem Siedepunkt vollständig in flüssiger Form vorliegt. Maßgeblich für das erfindungsgemäße Verfahren ist die Vermeidung von Feststoffablagerungen bei der Kondensation, so dass als Schmelzpunkt im Sinne des erfindungsgemäßen Verfahrens bevorzugt die obere Grenze dieser Temperaturintervalle herangezogen wird, also 130 °C für 1,5-Naphthalindiisocyanat beziehungsweise 99 °C für p-Phenylendiisocyanat. Daraus ergibt sich, dass der Druck P1 in dem Verdampfungsraum des Verdampfer-Kondensators so gewählt ist, dass sich für das Kühlmedium eine Siedetemperatur ergibt, die bei der destillativen Reinigung von 1,5-Naphthalindiisocyanat bevorzugt nicht unterhalb von 125 °C und bei der destillativen Reinigung von p-Phenylendiisocyanat bevorzugt nicht unterhalb von 94 °C liegt. Da sich die Schmelztemperatur, anders als die Siedetemperatur, nur minimal in Abhängigkeit vom Druck ändert, kann diese Änderung hier vernachlässigt werden und die Schmelztemperatur unter Normalbedingungen herangezogen werden.

Im Zweifelsfall kann die "Schmelztemperatur des Isocyanats" im Rahmen der vorliegenden Erfindung vorzugsweise mittels einer Kapillarmethode mit hinreichender Genauigkeit bestimmt werden. Entsprechende Vorgehensweisen sind dem Fachmann geläufig. Eine pulverisierte Probe der Substanz wird dazu 2 mm bis 5 mm hoch in ein Schmelzpunktröhrchen gefüllt und verdichtet, z.B. indem das Schmelzpunktröhrchen durch ein Glasrohr auf eine feste Unterlage fallen gelassen wird. Aufgrund der hohen Reaktivität von Isocyanaten wird das Röhrchen am oberen Ende zugeschmolzen. Im Bereich der zu erwartenden Schmelztemperatur, die sich aus Literaturangaben oder einer zuvor durchgeführten Grobbestimmung ergibt, wird mit einer niedrigen Heizrate von maximal 1 K pro Minute vorgegangen. Abgelesen wird die Temperatur, bei der das letzte feste Teilchen in die flüssige Phase übergeht.

Bevorzugt bedeuten erfindungsgemäß die Ausdrücke "umfassend" oder "enthaltend" "im Wesentlichen bestehend aus" und besonders bevorzugt "bestehend aus".

"Im Bereich von ... bis ..." schließt nicht nur die zwischen den genannten Grenzwerten liegenden Zahlen ein, sondern auch die Grenzwerte selbst.

Druckangaben sind, soweit nicht explizit anders angegeben, als Absolutdrucke zu verstehen.

Gehaltsangaben organischer Verbindungen beziehen sich im Rahmen der vorliegenden Erfindung bevorzugt auf per Gaschromatographie bestimmte Gehalte. Die quantitative Auswertung von Gaschromatogrammen, gegebenenfalls mit Hilfe eines internen Standards, ist dem Fachmann bekannt.

Es versteht sich, dass der Ausdruck "umfassend die Schritte in der folgenden Reihenfolge" bedeutet, dass das Verfahren auch kontinuierlich geführt werden kann.

Die Isocyanat-Zusammensetzung aus Schritt (i) des erfindungsgemäßen Verfahrens enthält bevorzugt mindestens 80 Gew.-%, weiter bevorzugt mindestens 90 Gew.-%, besonders bevorzugt mindestens 95 Gew.-% des Isocyanats, also an 1,5-Naphthalindiisocyanat oder p-Phenylendiisocyanat, bezogen auf die Gesamtmasse der Isocyanat-Zusammensetzung, sowie bevorzugt bis 20 Gew.-%, weiter bevorzugt bis 10 Gew.-%, besonders bevorzugt bis zu 5 Gew.-% an Nebenkomponenten, die einen höheren Siedepunkt als das Isocyanat aufweisen, wie beispielsweise Oligomere oder Harnstoffe des Isocyanats.

Die Herstellung der Isocyanat-Zusammensetzungen, die in Schritt (i) bereitgestellt werden, kann grundsätzlich auf beliebigen Wegen erfolgen. Bevorzugt ist die Herstellung durch Umsetzung der entsprechenden Diamine oder von deren Salzen mit Phosgen, zum Beispiel wie in der WO 2014044699 A1 beschrieben, unter Erhalt eines Rohprodukts, welches dann unter Erhalt der Isocyanat-Zusammensetzung aus Schritt (i) des erfindungsgemäßen Verfahrens einer Vorreinigung unterzogen wird. Bevorzugt wird das in der Phosgenierungsreaktion hergestellte Rohprodukt vorgereinigt, indem die leichtsiedenden Nebenkomponenten Phosgen, Chlorwasserstoff, Lösungsmittel und/oder andere leichter als das Isocyanat siedende Verunreinigungen beispielsweise durch Destillation, bevorzugt in einer mehrstufigen Destillationssequenz, weitgehend abgetrennt werden, so dass der Gehalt an leichtsiedenden Nebenkomponenten höchstens 5 Gew.-%, bevorzugt höchstens 3 Gew.-% und besonders bevorzugt höchstens 1 Gew.-%, ganz besonders bevorzugt höchstens 0,3 Gew.-%, bezogen auf die Gesamtmasse der Isocyanat-Zusammensetzung, beträgt.

Die entsprechenden Diamine sind vorliegend 1,5-Diaminonaphthalin für das 1,5-Naphthalindiisocyanat beziehungsweise p-Phenylendiamin für das p-Phenylendiisocyanat.

Eine solche Isocyanat-Zusammensetzung wird dann destillativ gereinigt. Hierzu wird die Isocyanat-Zusammensetzung in einer Verdampfungseinrichtung als Teil einer Vakuumapparatur erhitzt, so dass ein Teil des enthaltenen 1,5-Naphthalindiisocyanats oder p-Phenylendiisocyanats verdampft und in die Gasphase übergeht. Die Verdampfung erfolgt im Fall von 1,5-Naphthalindiisocyanat vorzugsweise bei einem Druck im Bereich von 90 Pa bis 2.000 Pa, weiter bevorzugt im Bereich von 100 bis 1.500 Pa und besonders bevorzugt im Bereich von 110 Pa bis 1.000 Pa und im Fall von p-Phenylendiisocyanat vorzugsweise bei einem Druck im Bereich von 1.100 bis 22.000 Pa, weiter bevorzugt im Bereich von 1200 bis 20.000 Pa und besonders bevorzugt im Bereich von 1300 Pa bis 10.000 Pa. Der Druck im Verdampfer wird also für das zu destillierende Isocyanat angepasst, wobei der bevorzugte Druckbereich maßgeblich vom Phasenverhalten des entsprechenden Isocyanats abhängt. Die Untergrenze für den Druck im Verdampfer ergibt sich zweckmäßig aus dem Druck am Tripelpunkt des Diisocyanats. Unterhalb dieses Drucks existiert keine flüssige Phase, so dass ohne weitere Druckerhöhungsstufen zwischen der Verdampfung und der Kondensation die Kondensation in flüssiger Form nicht möglich ist. Naturgemäß führt ein höherer Druck in der Verdampfungseinrichtung zu einer höheren Verdampfungstemperatur für das Isocyanat und erhöht somit die thermische Belastung, was wiederum eine stärkere Zersetzung und Nebenproduktbildung verursacht. Deshalb ist ein eher niedriger Druck zu bevorzugen. Ein zu niedriger Druck hingegen bewirkt bei Abwesenheit einer Druckerhöhungseinrichtung zwischen Verdampfungseinrichtung und Verdampfer-Kondensator auch einen niedrigeren Druck P1 und erschwert so die Kondensation des verdampften Isocyanats bis hin zur unerwünschten Ablagerung von ausgefrorenen Feststoffen. Je nach Leistungsfähigkeit des verwendeten Vakuumsystems und unter Berücksichtigung weiterer Faktoren wie Druckverlusten, Dichtigkeit der Destillationsapparatur, Inertgasanteil in der Vakuumapparatur und Dampfdruck der Isocyanat-Zusammensetzung ist häufig keine weitere über das Vakuumsystem selbst hinausgehende Regelung des Drucks nötig, da der Druck im System mit vertretbarem technischen Aufwand nicht beliebig weit abgesenkt werden kann und die zuvor genannten Untergrenzen auch bei voller Ausnutzung des Vakuumsystems nicht unterschritten werden. Dies gilt vor allem für die Destillation von Isocyanat-Zusammensetzungen, enthaltend 1,5-Naphthalindiisocyanat.

Der Druck P2 liegt für 1,5-Naphthalindiisocyanat im Bereich von 80 Pa bis 1.000 Pa, bevorzugt im Bereich von 90 Pa bis 500 Pa und besonders bevorzugt im Bereich von 100 Pa bis 400 Pa. Für p-Phenylendiisocyanat liegt der Druck P2 im Bereich von 1.000 Pa bis 20.000 Pa, bevorzugt im Bereich von 1.200 Pa bis 10.000 Pa und besonders bevorzugt im Bereich von 1.400 Pa bis 8.000 Pa.

Vorzugsweise wird der Druck P2 möglichst konstant gehalten. Es ist jedoch auch möglich, den Druck P2 im Laufe der Destillation zu erhöhen, um die Kondensationstemperatur des Isocyanats anzuheben. Dieses kann gemacht werden, um z.B. auf eine Verschlechterung des Wärmeübergangs im Kondensator zu reagieren. Vorzugsweise erfolgt eine solche Erhöhung nur für kurze Zeiträume von höchstens 6 h. Die höhere Kondensationstemperatur ermöglicht wiederum auch eine Erhöhung des Drucks P1 und damit der Verdampfungstemperatur des Kühlmediums im Verdampfungsraum des Verdampfer-Kondensators.

Das erfindungsgemäße Verfahren wird bevorzugt kontinuierlich durchgeführt. Entsprechend ist die Vorrichtung bevorzugt so gestaltet, dass das Verfahren kontinuierlich durchgeführt werden kann.

Als Apparate für das zumindest teilweise Verdampfen des in der Isocyanat-Zusammensetzung enthaltenen Isocyanats eignen sich alle gängigen Verdampfer-Apparate, die für den Betrieb im entsprechenden Unterdruck geeignet sind. Bevorzugt erfolgt das Verdampfen unter Verwendung eines Fallfilmverdampfers. Der nicht verdampfte Rückstand wird dabei vorzugsweise zurückgeführt und vor oder beim Eintritt in den Verdampfer mit der frisch eintretenden Isocyanat-Zusammensetzung vermischt. Durch diesen Kreislauf kann sichergestellt werden, dass die Verweilzeit der Isocyanat-Zusammensetzung auf den heißen Oberflächen des Verdampfers reduziert wird und die Benetzung der Oberflächen optimal ist. Dies bewirkt zusammen mit der höheren Strömungsgeschwindigkeit einen verbesserten Wärmeübergang, so dass die Heiztemperatur bzw. die Überhitzung der Verdampferoberflächen möglichst gering ausfällt. Ein Teil des nicht verdampften Rückstands kann periodisch oder kontinuierlich aus dem Kreislauf ausgeschleust werden, um eine zu starke Anreicherung von schwersiedenden Rückstandskomponenten zu verhindern. Optional kann aus diesem ausgeschleusten Rückstand in einer anderen Vorrichtung noch nicht abgetrenntes monomeres Isocyanat zurückgewonnen werden und entweder direkt als Produkt verwendet oder weiter gereinigt werden, z.B., indem es in Schritt (i) des hier beschriebenen Verfahrens im Rahmen der Bereitstellung der Isocyanat-Zusammensetzung dieser beigemischt wird.

Das verdampfte Isocyanat wird zum Verdampfer-Kondensator geleitet, wobei durch Wahl geeigneter Rohrleitungen und Apparate für einen geringen Druckverlust zwischen Verdampfer und Verdampfer-Kondensator gesorgt wird. Bevorzugt beträgt der Druckverlust weniger als 1.000 Pa, weiter bevorzugt weniger als 500 Pa, besonders bevorzugt weniger als 200 Pa, ganz besonders bevorzugt weniger als 100 Pa und am meisten bevorzugt weniger als 50 Pa. Der höhere Druck liegt dabei im Verdampfer vor, so dass das Gas ohne weitere Fördereinrichtung zum Verdampfer-Kondensator strömt. Im Kondensationsraum des Verdampfer-Kondensators wird das verdampfte Isocyanat zumindest teilweise, bevorzugt weitgehend, in flüssiger Form kondensiert. Unter "weitgehend" ist vorliegend zu verstehen, dass mindestens 80 Gew.-%, bevorzugt mindestens 90 Gew.-% und besonders bevorzugt mindestens 95 Gew.-% des im Gasstrom enthaltenen verdampften Isocyanats kondensiert werden. Die Kondensationswärme wird dabei an das Kühlmedium im Verdampfungsraum des Verdampfer-Kondensators abgegeben. Geeignete Kühlmedien sind beispielsweise Wasser oder organische Lösungsmittel mit Siedepunkten bei Normaldruck, also 1013,25 hPa, unterhalb von 200 °C. Besonders geeignete Kühlmedien sind Wasser oder Chlorbenzol, ganz besonders geeignet ist Wasser. Das flüssig kondensierte Isocyanat kann über eine Entnahmevorrichtung aus der Destillationsvorrichtung entnommen und dann nach Wunsch konfektioniert werden. Bevorzugt wird es auf einer gekühlten Walze kristallisiert und weiter bevorzugt in Form kleiner Schuppen von dieser Walze abgeschabt. Bei Bedarf können die Schuppen anschließend weiter klassiert, gesiebt, gesichtet oder zerkleinert werden.

Über den Druck P1 im Verdampfungsraum kann Einfluss auf die Siedetemperatur des Kühlmediums genommen werden. Der Druck wird dabei erfindungsgemäß mit Hilfe einer Druckanpassungseinrichtung so eingestellt, dass sich für das Kühlmedium eine Siedetemperatur ergibt, die mindestens 3 K unterhalb der Kondensationstemperatur des zu reinigenden Isocyanats unter den im Kondensationsraum herrschenden Bedingungen, jedoch nicht mehr als 5 K unterhalb der Schmelztemperatur, bevorzugt oberhalb der Schmelztemperatur des zu reinigenden Isocyanats bei Normaldruck liegt. Bevorzugt wird der Druck P1 so gewählt, dass sich eine Siedetemperatur für das Kühlmedium ergibt, die wenigstens 8 K unterhalb der Kondensationstemperatur des zu reinigenden Isocyanats unter den im Kondensationsraum herrschenden Bedingungen, dabei aber oberhalb von 130 °C bei der destillativen Reinigung von 1,5-Naphthalindiisocyanat oder oberhalb von 99 °C bei der destillativen Reinigung von p-Phenylendiisocyanat liegt. Besonders bevorzugt wird der Druck P1 so eingestellt, dass sich eine Siedetemperatur für das Kühlmedium ergibt, die mindestens 9 K unterhalb der Kondensationstemperatur des zu reinigenden Isocyanats unter den im Kondensationsraum herrschenden Bedingungen, dabei aber oberhalb von 132 °C bei der destillativen Reinigung von 1,5-Naphthalindiisocyanat oder oberhalb von 102 °C bei der destillativen Reinigung von p-Phenylendiisocyanat liegt.

Die Druckanpassungseinrichtung umfasst bevorzugt eine Steuerungs- oder Regelungseinheit, weiter bevorzugt eine Regelungseinheit, die auf wenigstens einen Aktor wirkt, der den Druck im Verdampfungsraum des Verdampfer-Kondensators direkt oder indirekt beeinflusst. Eine Steuerungseinheit wirkt dabei vorliegend über einen offenen Wirkungsablauf auf den Aktor, während eine Regelungseinheit einen gemessenen Istwert, beispielsweise für den Druck P1 oder die Temperatur im Verdampfungsraum des Verdampfer-Kondensators, mit einem vorgegebenen Sollwert vergleicht und bei Abweichungen die Stellgröße für den wenigstens einen Aktor nachjustiert, so dass ein geschlossener Regelkreis vorliegt. Bei dem Istwert, der für das Feedback verwendet wird, muss es sich dabei nicht zwangsläufig um den Druck selbst handeln. Alternativ kann beispielsweise auch die Temperatur im Verdampfungsraum des Verdampfer-Kondensators oder eine andere Messgröße verwendet werden, die mit dem Druck, zum Beispiel über thermodynamische Zusammenhänge, korreliert.

Der Aktor ist bevorzugt als Regelungsventil ausgestaltet, über das Gas oder Kühlflüssigkeit in den Verdampfungsraum des Verdampfer-Kondensators eingebracht oder aus diesem entfernt wird. Alternativ und insbesondere dann, wenn eine Rückkondensation des verdampften Kühlmediums innerhalb eines geschlossenen Systems mit der Verdampfung des Kühlmediums erfolgt, steuert der Aktor beispielsweise auch die Kühlleistung des Rückkondensators oder regelt diese, wobei eine Regelung bevorzugt ist, um so den Druck im Verdampfungsraum des Verdampfungskondensators anzupassen. Hierfür kann beispielsweise der Durchfluss an Kühlmittel durch den Rückkondensator oder die Temperatur des Kühlmittels geändert werden.

Die Einstellung des Drucks mit Hilfe der Druckanpassungseinrichtung ermöglicht eine präzise Einstellung der Siedetemperatur des Kühlmediums und damit eine optimale Kondensation des Isocyanats in flüssiger Form. Besonders vorteilhaft ist dabei, dass diese optimale Kondensation weitgehend unabhängig von der Art des im Verdampfer-Kondensator verwendeten Kühlmediums erreicht wird. So kann dieses anhand anderer Kriterien, wie z.B. der spezifischen Verdampfungsenthalpie, der Toxizität, der Verfügbarkeit, der Reaktivität und Verträglichkeit gegenüber anderen im Prozess vorkommenden Substanzen, des Schmelzpunkts oder der Viskosität ausgewählt werden, während man ohne die Druckanpassung auf ein Kühlmedium festgelegt wäre, das bei Umgebungsdruck einen Siedepunkt genau im richtigen Temperaturbereich aufweist. Außerdem ist von Vorteil, dass die optimalen Kondensationsbedingungen auch bei Veränderungen, z.B. des Wärmeübergangs oder der Gaszusammensetzung im Kondensationsraum des Verdampfer-Kondensators, aufrechterhalten werden können, da durch eine Anpassung des Drucks flexibel darauf reagiert werden kann. Der Einfluss des Umgebungsdrucks auf den Prozess wird auf diese Weise ebenfalls eliminiert.

Die Kondensationstemperatur des zu reinigenden Isocyanats lässt sich anhand der Dampfdruckkurven des Isocyanats und des Drucks P2 ermitteln. Dabei ist gegebenenfalls der Partialdruck von anwesenden Inertgasen im Kondensationsraum des Verdampfer-Kondensators zu berücksichtigen. Sofern aus der Literatur keine Dampfdruckkurve vorliegt, kann diese experimentell bestimmt werden, indem z.B. für verschiedene Temperaturen der Dampfdruck des Isocyanats gemäß der statischen Methode der Prüfrichtlinie OECD 104 "Dampfdruck" (2006) bestimmt wird. Bei unbekannter Zusammensetzung der Gasphase im Kondensationsraum des Verdampfer-Kondensators ist es auch möglich, die Kondensationstemperatur direkt im Kondensationsraum des Verdampfer-Kondensators experimentell zu bestimmen.

Beispielhaft seien hier einige Richtwerte für den Druck P1 im Verdampfungsraum des Verdampfer-Kondensators genannt. Wird als Kühlmedium Wasser verwendet und handelt es sich bei dem zu reinigenden Isocyanat um 1,5-Naphthalindiisocyanat, so liegt der Druck P1 im Verdampfungsraum des Verdampfer-Kondensators vorzugsweise im Bereich von 0,24 MPa bis 0,90 MPa, besonders bevorzugt im Bereich von 0,25 bis 0,65 MPa, ganz besonders bevorzugt im Bereich von 0,27 MPa bis 0,60 MPa, äußerst bevorzugt im Bereich von 0,3 MPa bis 0,55 MPa. Handelt es sich hingegen bei dem zu reinigenden Isocyanat um p-Phenylendiisocyanat und beim Kühlmedium um Wasser, so liegt der Druck P1 im Verdampfungsraum des Verdampfer-Kondensators vorzugsweise im Bereich von 0,085 MPa bis 0,90 MPa, besonders bevorzugt im Bereich von 0,10 MPa bis 0,65 MPa und ganz besonders bevorzugt im Bereich von 0,20 MPa bis 0,60 MPa. Dabei gilt für das vorliegende Verfahren weiterhin die Randbedingung, dass der Druck P1 so gewählt sein muss, dass die Siedetemperatur des Kühlmediums im Verdampfungsraum mindestens 3 K, vorzugsweise mindestens 8 K, besonders bevorzugt mindestens 9 K unterhalb der Kondensationstemperatur des zu reinigenden Isocyanats im Kondensationsraum des Verdampfer-Kondensators liegt. Folglich geht mit der Auswahl eines niedrigen Drucks P2 auch eine eher niedrige Obergrenze für den Druck P1 einher.

Wird hingegen als Kühlmedium Chlorbenzol verwendet und handelt es sich bei dem zu reinigenden Isocyanat um 1,5-Naphthalindiisocyanat, so liegt der Druck P1 im Verdampfungsraum des Verdampfer-Kondensators vorzugsweise im Bereich von 0,085 MPa bis 0,29 MPa, besonders bevorzugt im Bereich von 0,1 bis 0,25 MPa und ganz besonders bevorzugt im Bereich von 0,12 MPa bis 0,20 MPa. Handelt es sich hingegen bei dem zu reinigenden Isocyanat um p-Phenylendiisocyanat und beim Kühlmedium um Chlorbenzol, so liegt der Druck P1 im Verdampfungsraum des Verdampfer-Kondensators vorzugsweise im Bereich von 0,032 MPa bis 0,29 MPa, besonders bevorzugt im Bereich von 0,035 MPa bis 0,25 MPa und ganz besonders bevorzugt im Bereich von 0,04 MPa bis 0,2 MPa. Dabei gilt für das vorliegende Verfahren die Randbedingung, dass der Druck P1 so gewählt sein muss, dass die Siedetemperatur des Kühlmediums im Verdampfungsraum mindestens 3 K, vorzugsweise mindestens 8 K, besonders bevorzugt mindestens 9 K unterhalb der Kondensationstemperatur des zu reinigenden Isocyanats im Kondensationsraum des Verdampfer-Kondensators liegt. Folglich geht mit der Auswahl eines niedrigen Drucks P2 auch eine eher niedrige Obergrenze für den Druck P1 einher.

Besonders bevorzugt umfasst die Druckanpassungseinrichtung eine Regelungseinheit, die als Split-Range-Regelung ausgeführt ist, die den Druck P1 im Verdampfungsraum des Verdampfer-Kondensators einstellt. Dies hat den Vorteil einer genauen und zuverlässigen Druckregelung, die auf verschiedenartige Prozessschwankungen effizient reagieren kann und für eine gleichbleibende Siedetemperatur sorgt. Die Split-Range-Regelung kann beispielsweise durch eine Kombination aus einem Ventil für eine Inertgas- oder Dampfzufuhr, vorzugsweise Inertgaszufuhr, und einem Ventil für die Ableitung von Abgas realisiert werden. Bevorzugtes Inertgas ist Stickstoff. Ein Regler kann dann beide Ventile in einem festgelegten Bereich ansteuern, wobei üblicherweise das eine Ventil öffnet, während das andere schließt. Dies ermöglicht eine präzise Druckregelung und einen sicheren und effizienten Betrieb über einen weiten Bereich von Betriebsbedingungen, von Unterdruck bis Überdruck. Es ist bevorzugt, den Druck P1 höher zu wählen als den Druck P2, so dass im Falle einer Innenleckage des Verdampfer-Kondensators eher Kühlmedium auf die Prozessseite eindringt als umgekehrt Isocyanat in das Kühlsystem gelangt. Voraussetzung für ein solches bevorzugtes Druckgefälle im Rahmen des erfindungsgemäßen Verfahrens ist die Verwendung eines Kühlmediums, dessen Dampfdruckkurve oberhalb der des Isocyanats verläuft.

Die Kondensationswärme wird im Verdampfer-Kondensator an das Kühlmedium im Verdampfungsraum übertragen. Sofern dieses noch nicht die Siedetemperatur erreicht hat, erwärmt es sich hierdurch bis zur Siedetemperatur, bei der dann Teile des Kühlmediums in die Gasphase übergehen. Im stationären Betrieb stellt sich dann druckabhängig eine weitgehend konstante Temperatur des Kühlmediums ein. Kleinere Temperaturunterschiede ergeben sich beispielsweise ortsabhängig durch Unterschiede im statischen Druck der Flüssigkeitssäule. Ein Teil oder die Gesamtmenge des verdampften Kühlmediums kann über eine Druckhalteeinrichtung aus dem Verdampfungsraum ausgeschleust und zu einem Auslass geleitet werden. Sofern es für die gewünschte Temperatur nötig ist, einen Druck unterhalb des Atmosphärendrucks einzustellen oder einzuregeln, ist es zweckmäßig, diesen Auslass für das ausgeschleuste Kühlmedium an ein Vakuumsystem anzuschließen. Vorzugsweise handelt es sich bei der Druckhalteeinrichtung um einen Teil einer Split-Range-Regelung. Nach dem Verlassen des Verdampfer-Kondensators wird das verdampfte Kühlmedium selbst kondensiert und kann beispielsweise zur weiteren Verwendung in den Verdampfungsraum des Verdampfer-Kondensators zurückgeleitet oder -gefördert werden, wo es wieder in Kontakt mit der Wärmeübertragungsfläche kommt.

Gegebenenfalls kann ein weiterer Teil des verdampften Kühlmediums oder das gesamte verdampfte Kühlmedium auch gleich innerhalb des Verdampfer-Kondensators durch Kondensation an einer kalten Oberfläche, also an einem Rückkondensator, niedergeschlagen werden und dann frei ablaufend in den Verdampfungsraum des Verdampfer-Kondensators zurückfließen und dort erneut verdampfen. Die Rückkondensation kann an dieser Stelle problemlos durch indirekte Kühlung mit einem Kühlmedium erfolgen. Eine Verdampfungskühlung ist nicht erforderlich, da anders als bei der Kondensation des Isocyanats hier ein größeres Temperaturfenster zur Verfügung steht. Eine Verfestigung ist in der Regel nicht zu befürchten und es können größere Temperaturgradienten an der Kühlfläche akzeptiert werden. Die Kühlung kann letztlich einfach durch einen Sekundärkreislauf mit einem Kühlmedium, beispielsweise Kühlwasser, erfolgen.

Bevorzugt wird das gesamte im Verdampfungsraum des Verdampfer-Kondensators verdampfte Kühlmedium an einem Rückkondensator kondensiert und gelangt zurück in den Verdampfungsraum. Auf diese Weise lässt sich ein geschlossenes System aufbauen, welches abgesehen von einer anfänglichen Befüllung keine Zufuhr oder Entnahme von Kühlmedium erfordert. Der Druck P1 und damit die Temperatur im Verdampfungsraum des Verdampfer-Kondensators kann dann einfach über die Kühlleistung des Rückkondensators angepasst, vorzugsweise geregelt werden. Hierzu ist es zweckmäßig, eine Druck- und/oder eine Temperaturmessung im Verdampfungsraum des Verdampfer-Kondensators zu installieren, die dann auf den Durchfluss an Kühlmittel im Rückkondensator wirkt, beispielsweise über ein Regelventil in der Zu- oder Ableitung des Kühlmediums, vorzugsweise in der Ableitung des Kühlmediums.

Nicht im Kondensationsraum des Verdampfer-Kondensators kondensierte Anteile des verdampften Isocyanats können als nicht kondensierter Gasstrom aus dem Kondensationsraum des Verdampfer-Kondensators entnommen und weiter behandelt werden, um das in dem Gasstrom enthaltene Isocyanat zumindest teilweise zu entfernen. Dies kann beispielsweise erfolgen, indem der nicht kondensierte Gasstrom einer Gaswäsche mit einem inerten Lösungsmittel unterzogen wird. Vorzugsweise enthält dabei das Lösungsmittel ein halogeniertes aromatisches Lösungsmittel oder besteht, abgesehen von üblichen Verunreinigungen, daraus. Besonders bevorzugt enthält das Lösungsmittel Chlorbenzol, etwa in Mischung mit Dichlorbenzol, oder besteht daraus. Das Isocyanat wird dann in dem Chlorbenzol oder sonstigen Lösungsmittel gelöst und kann aus diesem wieder isoliert werden. Es versteht sich, dass die Lösungsmittel übliche Verunreinigungen enthalten können.

Alternativ erfolgt die zumindest teilweise Entfernung des Isocyanats aus dem nicht kondensierten Gasstrom durch Abscheidung als Feststoff in einer Nachkondensationsvorrichtung, umfassend wenigstens einen temperierbaren Nachkondensator, wobei dieser Nachkondensator in vorbestimmten Zeitintervallen oder bei Erreichen einer vorbestimmten Menge an abgeschiedenem Isocyanat aufgeheizt wird, um abgeschiedenes Isocyanat abzuschmelzen und aus dem Nachkondensator zu entfernen. Vorzugsweise ist der Nachkondensator mit einem metallischen Filter ausgestattet, durch das der nicht kondensierte Gasstrom geleitet wird. Durch die große Oberfläche wird die Abscheidung des Isocyanats optimiert. Vorteilhaft an diesem Verfahren ist, dass das aus dem nicht kondensierten Gasstrom entfernte Isocyanat nicht in einem Absorbens anfällt, sondern allenfalls geringe Anteile an niedrig siedenden Verunreinigungen enthält. Ist der Gehalt an solchen Verunreinigungen hinreichend niedrig, kann dieses Isocyanat mit der Hauptmenge an destilliertem Isocyanat, die im Verdampfer-Kondensator kondensiert wurde, verschnitten werden. Dabei sind gegebenenfalls vorhandene Spezifikationsgrenzen für niedrigsiedende Nebenkomponenten zu beachten. Alternativ kann dieses Isocyanat einer weiteren Aufreinigung, beispielsweise umfassend eine Extraktion, Destillation, Umkristallisation oder Heißfiltration, unterzogen werden. Für einen besonders reibungslosen Betrieb des Verfahrens ist es vorteilhaft, wenn die Nachkondensationsvorrichtung wenigstens zwei temperierbare Nachkondensatoren umfasst, die parallel installiert sind, wobei die Nachkondensationsvorrichtung so betrieben wird, dass wenigstens ein Nachkondensator weiter mit dem nicht kondensierten Gasstrom beaufschlagt wird, während wenigstens ein weiterer Nachkondensator abgeschmolzen und dabei vorzugsweise nicht mit dem nicht kondensierten Gasstrom beaufschlagt wird. Besonders bevorzugt umfasst die Nachkondensationsvorrichtung genau zwei parallel installierte Nachkondensatoren. Unter paralleler Installation ist vorliegend zu verstehen, dass die Apparate jeweils über eine eigene Zuleitung verfügen, über die sie mit einem Gasstrom aus dem Verdampfer-Kondensator beschickt werden können, sowie jeweils eine eigene Ableitung durch die das behandelte Gas zum Vakuum- oder Abgassystem abgeführt werden kann. Anders als bei einer seriellen Installation wird bei der parallelen Installation also nicht das Abgas aus dem einen Nachkondensator in einen weiteren Nachkondensator geleitet. Vorzugsweise sind die parallel installierten Nachkondensatoren individuell, also getrennt voneinander regel- und absperrbar.

In einer weiteren Ausführungsform der Erfindung erfolgt eine Überwachung des Wärmeübergangskoeffizienten im Verdampfer-Kondensator. Dies kann beispielsweise anhand eines computerimplementierten Modells erfolgen, welches Prozessparameter wie beispielsweise Massenströme, Drucke und Temperaturen verschiedener Prozessströme einliest und mit bekannten Stoffdaten verknüpft, um einen Wärmeübergangskoeffizienten für den Verdampfer-Kondensator zu errechnen. Ändert sich dieser Wert, kann entweder ein Hinweis über diese Änderung an einen Anlagenfahrer ausgegeben werden oder es können automatisiert Stellgrößen für bestimmte Prozessvariablen verändert werden. So ist es beispielsweise möglich, bei einer Verschlechterung des Wärmeübergangs, die auf Ablagerungen an den Wärmeübertragungsflächen hinweist, den Druck im Verdampfungsraum des Verdampfer-Kondensators und damit die Betriebstemperatur zu senken, so dass durch eine größere Temperaturdifferenz zwischen dem Kondensationsraum und dem Verdampfungsraum die Kühlleistung konstant gehalten werden kann. Sofern es sich bei Ablagerungen an den Wärmeübertragungsflächen lediglich um kristallisiertes Isocyanat handelt, kann alternativ der Druck im Verdampfungsraum temporär oder dauerhaft angehoben werden, um die Betriebstemperatur zu erhöhen und so das kristallisierte Isocyanat von den Wärmeübertragungsflächen abzuschmelzen. Alternativ zu der Ermittlung des Wärmeübergangskoeffizienten kann gegebenenfalls auch aus der Leistungsaufnahme eines gegebenenfalls vorhandenen Nachkondensators auf den Zustand des Verdampfer-Kondensators rückgeschlossen werden. Auch hier kann die Leistungsaufnahme des Nachkondensators mit Hilfe eines computerimplementierten Modells errechnet werden.

Ein weiterer Gegenstand der Erfindung ist eine Vorrichtung zur destillativen Reinigung von Isocyanaten, umfassend
a. eine Verdampfungseinrichtung zum zumindest teilweisen Verdampfen eines in einer Isocyanat-Zusammensetzung enthaltenen Isocyanats,
b. einen Verdampfer-Kondensator, der einen Kondensationsraum zur Kondensation des in den gasförmigen Zustand überführten Isocyanats sowie einen mit dem Kondensationsraum im Wärmeaustausch stehenden Verdampfungsraum zur Verdampfung eines Kühlmediums umfasst,
c. eine Vakuumerzeugungseinrichtung,
d. eine erste Druckanpassungseinrichtung, die dazu bestimmt ist, in dem wenigstens einen Verdampfungsraum einen Druck P1 einzustellen, bei dem sich für das Kühlmedium eine Siedetemperatur ergibt, die mindestens 3 K unterhalb der Kondensationstemperatur des zu reinigenden Isocyanats unter den im Kondensationsraum herrschenden Bedingungen, jedoch nicht mehr als 5 K unterhalb der Schmelztemperatur des zu reinigenden Isocyanats bei Normaldruck liegt,
e. eine Austragungsvorrichtung zum Austragen des flüssigen Isocyanat-Kondensats aus dem Kondensationsraum.

Die Vakuumerzeugungseinrichtung c, welche dazu ausgelegt ist, in der Verdampfungseinrichtung einen Unterdruck herzustellen, weist optional eine zweite Druckanpassungseinrichtung auf, die dazu ausgelegt ist, in dem wenigstens einen Kondensationsraum einen Druck P2 im Bereich von 80 Pa bis 20.000 Pa einzustellen. Dabei ist diese Druckanpassungseinrichtung nicht zwingend so ausgerichtet, dass mit ihr der gesamte Bereich über 80 Pa bis 20.000 Pa eingestellt werden kann, sondern es kann sich auch nur um einen Teilbereich innerhalb dieses Bereiches handeln, der bevorzugt die Realisierung der jeweiligen für 1,5-Naphthalindiisocyanat und p-Phenylendiisocyanat genannten Druckbereiche ermöglicht. Ohne eine solche Druckanpassungseinrichtung begrenzt sich das Vakuum im System von selbst, wobei der Druck dann insbesondere durch den Dampfdruck der Stoffe im System, die Leistungsfähigkeit der Vakuumerzeugungseinrichtung, Druckverluste in den Rohrleitungen sowie die Dichtigkeit der Vorrichtung bestimmt wird. Die zweite Druckanpassungseinrichtung erlaubt es, bei Bedarf einen höheren Druck als den, der sich durch die Vakuumerzeugungseinrichtung ergibt, vorzugeben und mit höherer Konstanz einzuhalten. Dies erleichtert die erfindungsgemäße Kondensation des Isocyanats in flüssiger Form, da der höhere Druck P2 zu einem größeren Temperaturfenster führt, in dem das Isocyanat als flüssige Phase vorliegt. Die zweite Druckanpassungseinrichtung umfasst bevorzugt eine Druckmesseinrichtung als Sensor zur Messung des Drucks P2. Bevorzugt handelt es sich bei dem Sensor um einen Membrandrucktransmitter. Als Aktor dient bevorzugt ein Regelventil, dass das Ausströmen von durch die Vakuumerzeugungseinrichtung angesaugtem Gas aus dem Kondensationsraum begrenzt und so auf den Druck P2 wirkt. Besonders bevorzugt wird der gemessene Druck im Sinne einer Regelung als Feedback-Signal verarbeitet, also mit einem Sollwert verglichen und bei einer Abweichung entsprechend der Stellwert für den Aktor automatisch angepasst (geschlossener Regelkreis).

Die Vorrichtung dient bevorzugt der Durchführung des erfindungsgemäßen Verfahrens und dies auch in sämtlichen Bevorzugungen und Ausführungsformen. Insbesondere ist der Verdampfer-Kondensator dafür eingerichtet, im Verdampfungsraum mit einem Druck im Bereich von 0,032 MPa bis 0,90 MPa, vorzugsweise 0,12 MPa bis 0,60 MPa betrieben zu werden.

Die Verdampfungseinrichtung umfasst vorzugsweise einen Verdampfer, beispielsweise einen Fallrohrverdampfer, Zwangsumlaufverdampfer, Plattenverdampfer oder Dünnschichtverdampfer, sowie die jeweils gegebenenfalls zugehörigen Peripheriegeräte, wie beispielsweise Pumpen, Entspannungsgefäße, Abscheider, Regelungs- und/oder Überwachungseinrichtungen. Die Auswahl einer geeigneten Verdampfungseinrichtung kann der Fachmann im Rahmen der Auslegung der Vorrichtung anhand ihm bekannter Kriterien wie beispielsweise Druckbedingungen, Durchsatz, Temperaturanforderungen und räumlichen Gegebenheiten treffen. Besonders bevorzugt umfasst die Verdampfungseinrichtung einen Fallrohrverdampfer mit einem vertikal angeordnetem Rohrbündel. Ganz besonders bevorzugt befindet sich am oberen Ende des Rohrbündels ein Verteiler, der dazu eingerichtet ist, die zu verdampfende Flüssigkeit auf die einzelnen, von außen beheizbaren Rohre zu verteilen.

Als Verdampfer-Kondensator eignen sich beispielsweise Rohrbündelwärmetauscher. Bevorzugt erfolgt die Kondensation dabei außerhalb der Rohre und das Verdampfen des Kühlmediums innerhalb der Rohre. Diese Anordnung ermöglicht einen geringen Druckverlust auf der Kondensationsseite und damit bei Bedarf einen niedrigeren Druck P2, was wiederum eine möglichst schonende Destillation ermöglicht.

Bei der Vakuumerzeugungseinrichtung handelt es sich beispielsweise um ein ein- oder mehrstufiges Vakuumsystem, umfassend wenigstens eine Vakuumpumpe. Als Vakuumpumpen können beispielsweise Flüssigkeitsringverdichter, Schraubenverdichter, Gasringpumpen, Gasstrahler, Kolbenpumpen oder Drehkolbengebläse eingesetzt werden. Bevorzugt handelt es sich bei der Vakuumerzeugungseinrichtung um ein wenigstens zweistufiges Vakuumsystem. Besonders bevorzugt umfasst das Vakuumsystem einen Flüssigkeitsringverdichter und einen oder mehrere Vorverdichter, wobei es sich bei dem oder den Vorverdichtern vorzugsweise um Gasstrahler, Schraubenverdichter, Gasringpumpen, Kolbenpumpen oder Drehkolbengebläse handelt. Zur Optimierung des Förderstroms kann beispielsweise ein Zwischenkondensator zwischen einem Vorverdichter und dem Flüssigkeitsringverdichter eingerichtet werden.

Die erste Druckregelungseinrichtung umfasst vorzugsweise wenigstens einen Aktor, der einen Prozessparameter beeinflusst, welcher sich auf den Druck in dem Verdampfungsraum des Verdampfer-Kondensators auswirkt. Weiter bevorzugt umfasst die Druckregelungseinrichtung einen Druck- und/oder einen Temperatursensor zur Messung des Drucks und/oder der Temperatur im Verdampfungsraum des Verdampfer-Kondensators und zwei Regelventile, wobei eines dazu eingerichtet ist, eine Inertgas- oder Dampfzufuhr in den Verdampfungsraum zu regeln und das andere dazu eingerichtet ist, den Abgasstrom aus dem Verdampfungsraum des Verdampfer-Kondensators zu regeln. Der Druck- und/oder Temperatursensor bildet in dieser Ausführungsform einen Regelkreis, der eingerichtet ist, den gewünschten Druck und damit die gewünschte Temperatur im Verdampfungsraum des Verdampfer-Kondensators einzustellen.

In einer bevorzugten Ausführungsform der erfindungsgemäßen Vorrichtung umfasst diese einen Rückkondensator, der dazu eingerichtet ist, verdampftes Kühlmedium zu kondensieren und flüssig in den Verdampfungsraum des Verdampfer-Kondensators zurückzuführen. In dieser Ausführungsform umfasst die erste Druckregelungseinrichtung vorzugsweise einen Druck- und/oder einen Temperatursensor zur Messung des Drucks und/oder der Temperatur im Verdampfungsraum des Verdampfer-Kondensators und ein Regelventil, dass auf den Kühlmitteldurchfluss des Rückkondensators wirkt. In dieser Ausführungsform bilden also der Druck- und/oder Temperatursensor und das Regelventil einen Regelkreis, der eingerichtet ist, den gewünschten Druck und damit die gewünschte Temperatur im Verdampfungsraum des Verdampfer-Kondensators einzustellen. Einen Abgasstrom oder eine Zugabe von beispielsweise Inertgas ist in diesem Fall nicht erforderlich, was die thermodynamischen Zusammenhänge vereinfacht.

Produktführende Rohrleitungen oder Behälter sind vorzugsweise beheizbar ausgelegt. Die Beheizung kann dabei beispielsweise mittels außen aufgebrachter elektrischer Heizelemente oder Dampfmäntel erfolgen.

Bevorzugt umfasst die erfindungsgemäße Vorrichtung eine Nachkondensationsvorrichtung, weiter bevorzugt umfassend wenigstens zwei parallel installierte Nachkondensatoren, die unabhängig voneinander dazu eingerichtet sind, von einem nicht kondensierten Gasstrom aus dem Kondensationsraum des Verdampfer-Kondensators durchströmt zu werden und nicht kondensierte Teile des Isocyanats abzuscheiden und die über eine Temperiereinrichtung verfügen, die dazu eingerichtet ist, den Nachkondensator auf eine Temperatur oberhalb der Schmelztemperatur des zu reinigenden Isocyanats zu erwärmen.

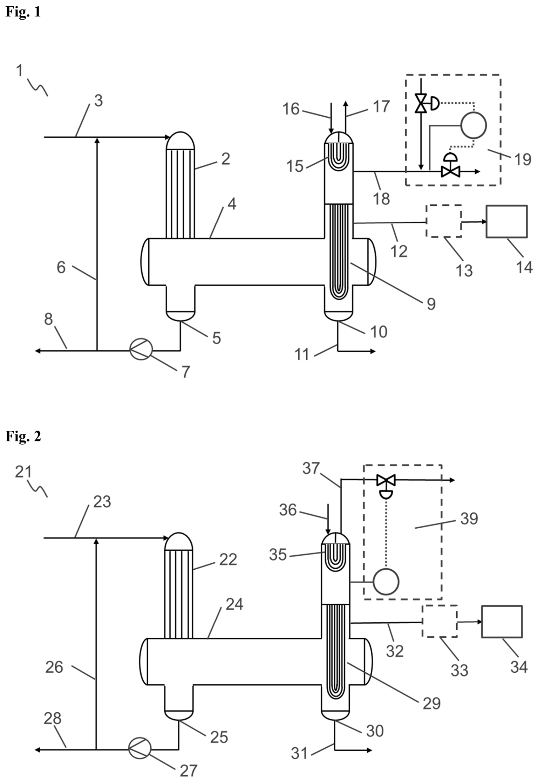

Die erfindungsgemäße Vorrichtung wird im Folgenden anhand von schematisch in Fig. 1 und Fig. 2 dargestellten Vorrichtungen erläutert, ohne jedoch dabei die Erfindung auf genau diese Ausführungsformen zu beschränken. Insbesondere weitere, nicht erfindungswesentliche Mess- und Regeleinrichtungen, aber auch Prozess- und Hilfseinrichtungen wie Befüll- und Entleerungsstutzen, Tropfenabscheider, Heizdampfleitungen, Probenahmestellen usw. wurden der Übersichtlichkeit halber nicht dargestellt.

Fig. 1 zeigt eine schematische Querschnittansicht einer erfindungsgemäßen Destillationsvorrichtung, die dazu eingerichtet ist, den Druck P1 über eine Split-Range-Regelung einzustellen. Die Vorrichtung 1 umfasst einen Verdampfer 2, eine mit dem Verdampfer 2 verbundene erste Zuleitung 3 zur Zuführung einer Isocyanat-Zusammensetzung, eine am gegenüberliegenden Ende des Verdampfers 2 befindlichen Entspannungsbehälter 4 mit einer ersten Entnahmeeinrichtung 5 für nicht verdampfte Anteile der Isocyanat-Zusammensetzung, eine Rezirkulationsleitung 6 mit einer Fördereinrichtung 7 für nicht verdampfte Anteile der Isocyanat-Zusammensetzung, eine erste Ableitung 8 zum periodischen oder kontinuierlichen Austragen nicht verdampfter Anteile der Isocyanat-Zusammensetzung (Rückstand), einen Verdampfer-Kondensator 9, eine zweite Entnahmeeinrichtung 10 für kondensiertes Isocyanat mit einer zweiten Ableitung 11 für kondensiertes Isocyanat (destilliertes Produkt), eine dritte Ableitung 12 zur Ableitung von nicht im Verdampfer-Kondensator 9 kondensiertem Gas (Abgas), gegebenenfalls durch eine Nachkondensationsvorrichtung 13 in eine Vakuumerzeugungsvorrichtung 14, einen Rückkondensator 15 für verdampftes Kühlmedium mit einer zweiten Zuleitung 16 und einer vierten Ableitung 17 für ein zweites Kühlmedium und eine über eine Verbindungsleitung 18 mit dem Verdampfungsraum des Verdampfer-Kondensators verbundene Split-Range-Druckregelungseinrichtung 19.

Fig. 2 zeigt eine schematische Querschnittansicht einer erfindungsgemäßen Destillationsvorrichtung, die dazu eingerichtet ist, den Druck P1 über eine Regelung der Rückkondensator-Leistung einzustellen. Die Vorrichtung 21 umfasst einen Verdampfer 22, eine mit dem Verdampfer 22 verbundene erste Zuleitung 23 zur Zuführung einer Isocyanat-Zusammensetzung, einen am gegenüberliegenden Ende des Verdampfers 22 befindlichen Entspannungsbehälter 24 mit einer ersten Entnahmeeinrichtung 25 für nicht verdampfte Anteile der Isocyanat-Zusammensetzung, eine Rezirkulationsleitung 26 mit einer Fördereinrichtung 27 für nicht verdampfte Anteile der Isocyanat-Zusammensetzung, eine erste Ableitung 28 zum periodischen oder kontinuierlichen Austragen nicht verdampfter Anteile der Isocyanat-Zusammensetzung (Rückstand), einen Verdampfer-Kondensator 29, eine zweite Entnahmeeinrichtung 30 für kondensiertes Isocyanat mit einer zweiten Ableitung 31 für kondensiertes Isocyanat (destilliertes Produkt), eine dritte Ableitung 32 zur Ableitung von nicht im Verdampfer-Kondensator 29 kondensiertem Gas (Abgas), gegebenenfalls durch eine Nachkondensationsvorrichtung 33 in eine Vakuumerzeugungsvorrichtung 34, einen Rückkondensator 35 für verdampftes Kühlmedium mit einer zweiten Zuleitung 36 und einer vierten Ableitung 37 für ein zweites Kühlmedium und eine Druckregelungseinrichtung 39.

### Beispiel 1

In die in Fig. 2 skizzierte Apparatur wird über die erste Zuleitung 23 aus einer Rohproduktvorlage eine Isocyanat-Zusammensetzung, enthaltend 96 Gew.-% 1,5-Naphthalindiisocyanat und 4 Gew.-% höher siedende Nebenkomponenten, kontinuierlich eingebracht. Der Druck in der Vorrichtung 21, gemessen im Kondensationsraum des Verdampfer-Kondensators 29, wird über die Vakuumerzeugungsvorrichtung 34 auf ca. 200 Pa gesenkt. Die Kondensationstemperatur des verdampften 1,5-Naphthalindiisocyanats unter diesen Bedingungen beträgt 145 °C. Die in die Vorrichtung 21 eingebrachte Isocyanat-Zusammensetzung wird über einen Verteiler auf die Rohre des Fallrohrverdampfers 22 verteilt, der mit Heizdampf (6 bar) betrieben wird, so dass aus der Zusammensetzung ein Teil des enthaltenen 1,5-Naphthalindiisocyanats verdampft. Gas und nicht verdampfte Flüssigkeit gelangen am unteren Ende des Fallrohrverdampfers 22 in den Entspannungsbehälter 24 und werden dort getrennt. Die nicht verdampfte Flüssigkeit wird mit an der Entnahmevorrichtung 25 des Entspannungsbehälters 24 entnommen und mit Hilfe einer Pumpe 27 zu einem Teil durch die Rezirkulationsleitung 26 in die Zuleitung 23 zurückgefördert, wo es mit frischer Isocyanat-Zusammensetzung vermischt wird. Ein weiterer Teil wird in periodischen Abständen zur weiteren Aufbereitung über die erste Ableitung 28 ausgeschleust.

Das Gas gelangt durch den Entspannungsbehälter zum Verdampfer-Kondensator 29 und wird dort an der Außenseite der Rohre kondensiert. In den Rohren befindet sich Wasser, das unter Aufnahme der übertragenen Kondensationswärme siedet. Über die die Druckanpassungseinrichtung, hier eine Druckregelungseinrichtung 39 wird im Verdampfungsraum ein Druck von 0,32 MPa eingestellt, so dass sich für das Wasser eine Siedetemperatur von 136 °C ergibt. Das im Verdampfungsraum des Verdampfer-Kondensators 29 verdampfte Wasser wird am Rückkondensator 35 kondensiert und läuft zurück in die Rohre des Verdampfer-Kondensators 29, wo es erneut zur Siedekühlung genutzt wird. Der Rückkondensator 35 wird mit Kühlwasser zurückgekühlt. Kondensiertes Isocyanat fällt flüssig an und fließt frei nach unten ab, wo es über die zweite Entnahmeeinrichtung 30 entnommen und durch die zweite Ableitung 31 zu einer Konfektionierungseinrichtung abgeführt wird. Ein Feststoffhandling innerhalb der Destillationsvorrichtung oder ein periodisches Abschmelzen des Kondensators ist nicht erforderlich.

Nicht kondensiertes Gas passiert einen Wäscher 33, wo es im Gegenstrom mit Chlorbenzol gewaschen wird, um 1,5-Naphthalindiisocyanat aus dem Gasstrom zurückzugewinnen.

Die in der vorangehenden Figurenbeschreibung genutzten Bezugszeichen sind der Übersichtlichkeit wegen auch in den Patentansprüchen genannt, deren Umfang sie nicht beschränken sollen.

### Beispiel 2

Auf die gleiche Art wie in Beispiel 1 wird eine Isocyanat-Zusammensetzung, enthaltend 97 Gew.-% p-Phenylendiisocyanat und 3 Gew.-% höher siedende Nebenkomponenten, destilliert. Dazu wird die gleiche Vorrichtung wie in Beispiel 1 verwendet, wobei der im Kondensationsraum des Verdampfer-Kondensators 29 gemessene Druck über die Vakuumerzeugungseinrichtung auf 3000 Pa und der Druck im Verdampfungsraum des Verdampfer-Kondensators 29 mit Hilfe der Druckregelungseinrichtung 39 auf einen Druck von 0,14 MPa eingestellt wird. Damit ergibt sich für das Isocyanat eine Kondensationstemperatur von 128 °C und für das Wasser im Verdampfungsraum eine Siedetemperatur von 109 °C. Es gelingt, das p-Phenylendiisocyanat in flüssiger Form zu kondensieren und über die zweite Entnahmeeinrichtung 30 zu entnehmen.

Ein Feststoffhandling innerhalb der Destillationsvorrichtung oder ein periodisches Abschmelzen des Kondensators ist nicht erforderlich.

Nicht kondensiertes Gas passiert einen Wäscher 33, wo es im Gegenstrom mit Chlorbenzol gewaschen wird, um p-Phenylendiisocyanat aus dem Gasstrom zurückzugewinnen.

### Diskussion der Beispiele

Die Beispiele zeigen die Vorteile des erfindungsgemäßen Verfahrens und der erfindungsgemäßen Vorrichtung. So können Isocyanate mit Schmelzpunkten im Bereich von 95 °C bis 160 °C destilliert werden, ohne dass im Verdampfer-Kondensator ein Wechsel des Kühlmediums erforderlich wird. Außerdem ist innerhalb der Destillationsvorrichtung weder ein Feststoffhandling noch ein periodisches Abschmelzen des Kondensators erforderlich, da das Isocyanat-Kondensat flüssig anfällt. Dabei wird durch die Siedekühlung ein optimaler Wärmeübergang sichergestellt, so dass die Wärmeübertragungsflächen klein gehalten werden können. Insbesondere kann so Wasser für eine Vielzahl von verschiedenen Isocyanaten mit verschiedenen Schmelz- und Siedepunkten eingesetzt werden, was aufgrund seiner besonders guten Wärmeübertragungseigenschaften, der hohen Verdampfungsenthalpie, der Ungiftigkeit und der guten Verfügbarkeit von besonderem Vorteil ist. Sollten sich die Kondensationsbedingungen z.B. durch eine Druckveränderung, die Anreicherung von Inertgasen oder ein leichtes Fouling des Verdampfer-Kondensators verändern, können diese Veränderungen einfach durch eine Änderung des über die Druckregelungseinrichtung 19 eingestellten Drucks im Verdampfungsraum des Verdampfer-Kondensators reagiert werden.

## Patentansprüche

1. Ein Verfahren zur destillativen Reinigung von 1,5-Naphthalindiisocyanat oder p-Phenylendiisocyanat, umfassend die Schritte
(i) Bereitstellen einer Isocyanat-Zusammensetzung, enthaltend 1,5-Naphthalindiisocyanat oder p-Phenylendiisocyanat,
(ii) zumindest teilweises Verdampfen des in der Isocyanat-Zusammensetzung enthaltenen 1,5-Naphthalindiisocyanats oder p-Phenylendiisocyanats unter Erhalt dieses Isocyanats im gasförmigen Zustand,
(iii) zumindest teilweise Kondensation und dadurch Erhalt eines Isocyanat-Kondensats des in Schritt (ii) in den gasförmigen Zustand überführten Isocyanats in einem Verdampfer-Kondensator (9; 29) unter Verwendung eines Kühlmediums, umfassend
a) wenigstens einen Kondensationsraum zur Kondensation des in Schritt (ii) in den gasförmigen Zustand überführten Isocyanats und
b) wenigstens einen mit dem Kondensationsraum im Wärmeaustausch stehenden Verdampfungsraum zur teilweisen Verdampfung des Kühlmediums,
(iv) Austragen des in Schritt (iii) erhaltenen flüssigen Isocyanat-Kondensats aus dem Kondensationsraum,
**dadurch gekennzeichnet,**
**dass** mit Hilfe einer Druckanpassungseinrichtung ein Druck P1 in dem wenigstens einen Verdampfungsraum eingestellt wird, bei dem sich für das Kühlmedium eine Siedetemperatur ergibt, die mindestens 3 K unterhalb der Kondensationstemperatur des zu reinigenden Isocyanats unter den im Kondensationsraum herrschenden Bedingungen, jedoch nicht mehr als 5 K unterhalb der Schmelztemperatur des zu reinigenden Isocyanats bei Normaldruck liegt,
wobei,
wenn es sich bei dem Isocyanat um 1,5-Naphthalindiisocyanat handelt, in dem wenigstens einen Kondensationsraum, in dem die Kondensation von gasförmigem Isocyanat aus Schritt (ii) erfolgt, ein Druck P2 im Bereich von 80 bis 1.000 Pa vorliegt,
und wobei,
wenn es sich bei dem Isocyanat um p-Phenylendiisocyanat handelt, in dem wenigstens einen Kondensationsraum, in dem die Kondensation von gasförmigem Isocyanat aus Schritt (ii) erfolgt, ein Druck P2 im Bereich von 1.000 bis 20.000 Pa vorliegt.

2. Verfahren gemäß Anspruch 1, **dadurch gekennzeichnet, dass** die Isocyanat-Zusammensetzung mindestens 80 Gew.-% des Isocyanats 1,5-Naphthalindiisocyanat oder p-Phenylendiisocyanat, bezogen auf die Gesamtmasse der Isocyanat-Zusammensetzung, enthält.

3. Verfahren gemäß Anspruch 1 oder 2, **dadurch gekennzeichnet, dass** der Druck P1 mit Hilfe einer Split-Range-Regelung eingestellt wird.

4. Verfahren gemäß einem der Ansprüche 1 bis 3, **dadurch gekennzeichnet, dass** das Kühlmedium Wasser oder Chlorbenzol, bevorzugt Wasser ist.

5. Verfahren gemäß einem der Ansprüche 1 bis 4, **dadurch gekennzeichnet, dass** das im Verdampfungsraum des Verdampfer-Kondensators (9; 29) verdampfte Kühlmedium entweder innerhalb des Verdampfungsraums des Verdampfer-Kondensators kondensiert wird oder an anderer Stelle kondensiert und in den Verdampfungsraum des Verdampfer-Kondensators zurückgeführt wird.

6. Verfahren gemäß einem der Ansprüche 1 bis 4, **dadurch gekennzeichnet, dass** das Kühlmedium Wasser ist und das verdampfte Wasser als Heizdampf in einem anderen Prozessschritt eingesetzt wird.

7. Verfahren gemäß einem der Ansprüche 1 bis 6, **dadurch gekennzeichnet, dass** das Kühlmedium Wasser und das zu reinigende Isocyanat 1,5-Naphthalindiisocyanat ist, wobei der Druck P1 im Verdampfungsraum des Verdampfer-Kondensators im Bereich von 0,24 MPa bis 0,90 MPa, bevorzugt im Bereich von 0,25 bis 0,65 MPa, besonders bevorzugt im Bereich von 0,27 MPa bis 0,60 MPa, ganz besonders bevorzugt im Bereich von 0,30 bis 0,55 MPa liegt,
oder
dass das Kühlmedium Wasser und das zu reinigende Isocyanat p-Phenylendiisocyanat ist, wobei der Druck P1 im Verdampfungsraum des Verdampfer-Kondensators im Bereich von 0,085 MPa bis 0,90 MPa, bevorzugt im Bereich von 0,10 MPa bis 0,65 MPa und besonders bevorzugt im Bereich von 0,20 MPa bis 0,60 MPa liegt.

8. Verfahren gemäß einem der Ansprüche 1 bis 7, **dadurch gekennzeichnet, dass** ein nicht kondensierter, Isocyanat-haltiger Gasstrom aus dem Kondensationsraum des Verdampfer-Kondensators (9; 29) entnommen wird und aus diesem das enthaltene Isocyanat zumindest teilweise entfernt wird.

9. Verfahren gemäß Anspruch 8, **dadurch gekennzeichnet, dass** die teilweise Entfernung des Isocyanats aus dem nicht kondensierten Gasstrom durch eine Gaswäsche mit einem inerten Lösungsmittel, vorzugsweise mit einem halogenierten aromatischen Lösungsmittel, besonders bevorzugt mit Chlorbenzol oder einer Mischung von Chlorbenzol mit Dichlorbenzol, erfolgt.

10. Verfahren gemäß Anspruch 8, **dadurch gekennzeichnet, dass** die teilweise Entfernung des Isocyanats aus dem nicht kondensierten Gasstrom durch Abscheidung als Feststoff in einer Nachkondensationsvorrichtung (13; 33), umfassend wenigstens einen temperierbaren Nachkondensator, erfolgt, wobei dieser Nachkondensator in vorbestimmten Zeitintervallen oder bei Erreichen einer vorbestimmten Menge an abgeschiedenem Isocyanat aufgeheizt wird, um abgeschiedenes Isocyanat abzuschmelzen und aus dem Nachkondensator zu entfernen.

11. Verfahren gemäß Anspruch 10, **dadurch gekennzeichnet, dass** die Nachkondensationsvorrichtung (13; 33) mindestens zwei temperierbare Nachkondensatoren umfasst, die parallel installiert sind, wobei die Nachkondensationsvorrichtung (13; 33) so betrieben wird, dass mindestens ein Nachkondensator weiter mit dem nicht kondensierten Gasstrom beaufschlagt wird, während mindestens ein Nachkondensator abgeschmolzen und dabei vorzugsweise nicht mit dem nicht kondensierten Gasstrom beaufschlagt wird.

12. Verfahren nach einem der vorhergehenden Ansprüche, wobei der Wärmeübergangskoeffizient im Verdampfer-Kondensator computerimplementiert überwacht wird, wobei anhand von Betriebsparametern ein Wärmeübergangskoeffizient errechnet wird und dieser bei Veränderungen oder Abweichungen von einem für den Verdampfer-Kondensator erwarteten Wärmeübergangskoeffizienten in mindestens eine neue Stellgröße für mindestens einen Aktor übersetzt wird, der den Wärmeübergang beeinflusst.

13. Vorrichtung (1; 21) zur destillativen Reinigung von Isocyanaten, umfassend
a. eine Verdampfungseinrichtung (2; 22) zum zumindest teilweisen Verdampfen eines in einer Isocyanat-Zusammensetzung enthaltenen Isocyanats,
b. einen Verdampfer-Kondensator (9; 29), der einen Kondensationsraum zur Kondensation des in den gasförmigen Zustand überführten Isocyanats sowie einen mit dem Kondensationsraum im Wärmeaustausch stehenden Verdampfungsraum zur Verdampfung eines Kühlmediums umfasst,
c. eine Vakuumerzeugungseinrichtung (34),
d. eine erste Druckanpassungseinrichtung (19; 39), die dazu bestimmt ist, in dem wenigstens einen Verdampfungsraum einen Druck P1 einzustellen, bei dem sich für das Kühlmedium eine Siedetemperatur ergibt, die mindestens 3 K unterhalb der Kondensationstemperatur des zu reinigenden Isocyanats unter den im Kondensationsraum herrschenden Bedingungen, jedoch nicht mehr als 5 K unterhalb der Schmelztemperatur des zu reinigenden Isocyanats bei Normaldruck liegt,
e. eine Austragungsvorrichtung (10, 11; 30, 31) zum Austragen des flüssigen Isocyanat-Kondensats aus dem Kondensationsraum.

14. Vorrichtung (1; 21) nach Anspruch 13, umfassend eine Nachkondensationsvorrichtung (13; 33), umfassend wenigstens zwei parallel installierte Nachkondensatoren, die unabhängig voneinander dazu eingerichtet sind, von einem nicht kondensierten Gasstrom aus dem Kondensationsraum des Verdampfer-Kondensators durchströmt zu werden und nicht kondensierte Teile des Isocyanats abzuscheiden und die über eine Temperiereinrichtung verfügen, die dazu eingerichtet ist, den Nachkondensator auf eine Temperatur oberhalb der Schmelztemperatur des zu reinigenden Isocyanats zu erwärmen.

15. Vorrichtung (1; 21) nach Anspruch 13 oder 14, wobei die Vakuumerzeugungseinrichtung (34) eine zweite Druckanpassungseinrichtung aufweist, die dazu ausgelegt ist, in dem wenigstens einen Kondensationsraum einen Druck P2 im Bereich von 80 Pa bis 20.000 Pa einzustellen.
